# EUROPÄISCHE PATENTANMELDUNG

(11) **EP 1 897 567 A1**
(43) Veröffentlichungstag der Anmeldung: **12.03.2008**
(21) Anmeldenummer: 07033506.2
(22) Anmeldetag: 10.07.2007
(51) Int. Cl.: A61L 27/50, A61L 31/18

(54) **Röntgenmarker für medizinische Implantate aus einem biokorrodierbaren metallischen Werkstoff**

(30) Priorität: 07.08.2006 DE 102006038238
(71) Anmelder: BIOTRONIK VI Patent AG, 6341 Baar (CH)
(72) Erfinder: Gerold, Bodo, Dr., 91225 Zellingen (DE); Riedmüller, Johannes, 90411 Nürnberg (DE); Müller, Heinz, Dr., 91052 Erlangen (DE); Harder, Claus, Dr., 91080 Uttenreuth (DE)
(74) Vertreter: Lindner-Vogt, Karin L.

(57) **Zusammenfassung**

Die Erfindung betrifft einen Röntgenmarker für medizinische Implantate aus einem biokorrodierbaren metallischen Werkstoff. Der Röntgenmarker zeichnet sich dadurch aus, dass er ein Borid oder Carbid der Elemente Tantal oder Wolfram ist.

## Beschreibung

Die Erfindung betrifft einen Röntgenmarker für medizinische Implantate aus einem biokorrodierbaren metallischen Werkstoff, ein medizinisches Implantat mit einem Röntgenmarker und eine neue Verwendung eines Borids oder Carbids der Elemente Tantal oder Wolfram für einen Röntgenmarker.

Implantate haben in vielfältiger Ausführungsform Anwendung in der modernen Medizintechnik gefunden. Sie dienen beispielsweise der Unterstützung von Gefäßen, Hohlorganen und Gangsystemen (Endovaskuläre Implantate), zur Befestigung und temporären Fixierung von Gewebeimplantaten und Gewebstransplantationen, aber auch zu orthopädischen Zwecken, zum Beispiel als Nagel, Platte oder Schraube.

Häufig ist nur eine temporäre Stütz- beziehungsweise Haltefunktion bis zum Abschluss des Heilungsprozesses notwendig beziehungsweise erwünscht. Um Komplikationen zu vermeiden, die aus dem dauerhaften Verbleib der Implantate im Körper resultieren, müssen die Implantate entweder wieder operativ entfernt werden oder sie bestehen aus einem Werkstoff, der allmählich im Körper abgebaut wird, das heißt biokorrodierbar ist. Die Zahl biokorrodierbarer Werkstoffe auf der Basis von Polymeren oder Legierungen ist stetig gewachsen. So sind unter anderen biokorrodierbare Metalllegierungen der Elemente Magnesium, Eisen und Wolfram bekannt.

EP 1 270 023 beschreibt eine Magnesiumlegierung, die sich für endovaskuläre und orthopädische Implantate eignet. Die Legierung kann bis zu 5 Gewichtsprozent seltene Erden enthalten.

Die aus dem Stand der Technik bekannten biodegradierbaren Metalllegierungen und Polymere für medizinische Implantate haben jedoch den Nachteil, dass sie nicht oder nur in einem nicht zufrieden stellenden Ausmaß im gängigen Röntgenverfahren sichtbar sind. Zur postoperativen Überwachung des Heilungsfortschrittes oder zur Kontrolle minimalinvasiver Eingriffe ist aber gerade die Röntgendiagnostik ein wichtiges Instrumentarium. So werden z. B. seit einigen Jahren Stents in den Koronararterien bei der akuten Myokardinfarkttherapie platziert. Der Stent wird im Bereich der Läsion der koronaren Gefäßwand positioniert und soll eine Obstruktion der Gefäßwand nach deren Aufweitung verhindern. Der Vorgang der Positionierung und Expansion der Stents muss während der Prozedur durch den Kardiologen laufend überwacht werden.

Die Röntgensichtbarkeit eines aus einem metallischen oder polymeren Werkstoff hergestellten Implantats hängt zum einen von der Materialdicke und zum anderen von dessen Röntgenabsorptionskoeffizienten ab. Letzterer ist wiederum abhängig vom Energiebereich der Röntgenstrahlung: im medizinischen Bereich liegt dieser in der Regel bei 60 bis 120 keV. Mit steigender Ordnungszahl im Periodensystem und steigender Dichte des Materials wird der Röntgenabsorptionskoeffizienten in der Regel größer.

Zur Verbesserung der Röntgensichtbarkeit werden die Implantate dementsprechend mit Markern versehen, z. B. in Form einer Beschichtung, eines Bandes, eines Inlays oder eines mit dem Implantat fest verbundenen Formkörpers aus dem radioopaken Material. Für die Wahl des Markers sollten in der Regel noch folgende Punkte beachtet werden: (i) die Funktionalität des Implantats darf nicht durch die Gegenwart des Röntgenmarkers eingeschränkt sein; (ii) der Marker muss biokompatibel sein; und (iii) der Marker muss derart mit dem Implantat verbunden sein, dass ein Verlust desselben bei der Implantation ausgeschlossen ist.

Für Implantate, die zum permanenten Verbleib im Körper des Patienten ausgelegt sind oder chirurgisch zu einem späteren Zeitpunkt wieder entfernt werden, erfüllen Edelmetalle, wie Gold und Platin, in der Regel die genannten Kriterien.

Bei Implantaten aus biokorrodierbaren metallischen Werkstoffen auf der Basis von Magnesium, Eisen oder Wolfram bestehen jedoch erhöhte Anforderungen an das Markermaterial:
- der Marker sollte durch die korrosiven Prozesse nicht frühzeitig vom Grundkörper des Implantates getrennt werden, um einer Fragmentbildung und damit der Gefahr der Embolisation vorzubeugen;
- der Marker sollte schon bei geringen Materialdicken eine hinreichende Röntgendichtigkeit besitzen; und
- das Markermaterial sollte möglichst keinen oder allenfalls einen geringen Einfluss auf die Degradation des Grundkörpers haben.

DE 103 61 942 A1 beschreibt einen radioopaken Marker für medizinische Implantate, der 10 bis 90 Gewichtsprozente einer biokorrodierbaren Basiskomponente, insbesondere aus der Gruppe der Elemente Magnesium, Eisen und Zink, enthält. Weiterhin enthält der Marker 10 bis 90 Gewichtsprozente eines oder mehrerer radioopaker Elemente aus der Gruppe 1, Au, Ta, Y, Nb, Mo, Ru, Rh, Ba, La, Ce, Pr, Nd, Sm, Eu, Gd, Tb, Dy, Ho, Er, Tm, Yb, Lu, Hf, Ta, W, Re, Os und Bi als Markerkomponente. Die beschriebenen Marker eignen sich demnach prinzipiell für den Einsatz in biokorrodierbaren Implantaten, insbesondere solchen aus biokorrodierbaren Magnesiumlegierungen.

Bei Einsatz von Markern aus metallischen Werkstoffen auf biokorrodierbaren metallischen Grundkörpern besteht jedoch das besondere Problem, dass sich aufgrund von elektrochemischen Wechselwirkungen zwischen den beiden metallischen Materialien die Degradation des Grundkörpers in einem Kontaktbereich zwischen Marker und Grundkörper ändert, d. h. in der Regel beschleunigt ist.

Der vorliegenden Erfindung liegt die Aufgabe zugrunde, einen Röntgenmarker für medizinische Implantate aus einem biokorrodierbaren metallischen Werkstoff bereitzustellen, der insbesondere das Auftreten unerwünschter elektrochemischer Wechselwirkungen mit dem metallischen Grundkörper des Implantats vermeidet.

Nach einem ersten Aspekt der Erfindung wird diese Aufgabe durch einen Röntgenmarker für medizinische Implantate aus einem biokorrodierbaren metallischen Werkstoff nach Anspruch 1 gelöst. Der Röntgenmarker zeichnet sich dadurch aus, dass er ein Borid oder Carbid der Elemente Tantal oder Wolfram ist. Es hat sich gezeigt, dass die hohe Dichte der genannten anorganischen Verbindungen und hohe Ordnungszahl seiner metallischen Komponenten eine für den Einsatz in der Medizintechnik hinreichende Röntgensichtbarkeit gewährt. Weiterhin wurde festgestellt, dass die anorganischen Salze in physiologischen Medien unlöslich und inert sind. Es ist daher von einer hohen Gewebsverträglichkeit auszugehen, auch wenn der Marker nach dem Abbau des Implantats im Körper verbleibt.

Die Begriffe Boride und Carbide sind Sammelbezeichnungen für Verbindungen aus Bor bzw. Kohlenstoff mit einem Metall, hier Tantal oder Wolfram. Die Boride und Carbide von Tantal und Wolfram können als nichtstöchiometrische Verbindungen mit Legierungscharakter vorliegen.

Vorzugsweise ist der biokorrodierbare metallische Werkstoff eine biokorrodierbare Legierung ausgewählt aus der Gruppe Magnesium, Eisen und Wolfram; insbesondere ist der biokorrodierbare metallische Werkstoff eine Magnesiumlegierung. Unter Legierung wird vorliegend ein metallisches Gefüge verstanden, deren Hauptkomponente Magnesium, Eisen oder Wolfram ist. Hauptkomponente ist die Legierungskomponente, deren Gewichtsanteil an der Legierung am höchsten ist. Ein Anteil der Hauptkomponente beträgt vorzugsweise mehr als 50 Gew.%, insbesondere mehr als 70 Gew.%.

Ist der Werkstoff eine Magnesiumlegierung, so enthält diese vorzugsweise Yttrium und weitere Seltenerdmetalle, da sich eine derartige Legierung aufgrund ihrer physikochemischen Eigenschaften und hohen Biokompatibilität, insbesondere auch seiner Abbauprodukte, auszeichnet.

Besonders bevorzugt wird eine Magnesiumlegierung der Zusammensetzung Seltenerdmetalle 5,2 - 9,9 Gew.%, davon Yttrium 3,7 - 5,5 Gew.%, und Rest < 1 Gew.%, wobei Magnesium den auf 100 Gew.% fehlenden Anteil an der Legierung einnimmt, eingesetzt. Diese Magnesiumlegierung bestätigte bereits experimentell und in ersten klinischen Versuchen ihre besondere Eignung, d. h. zeigt eine hohe Biokompatibilität, günstige Verarbeitungseigenschaften, gute mechanische Kennwerte und ein für die Einsatzzwecke adäquates Korrosionsverhalten. Unter der Sammelbezeichnung "Seltenerdmetalle" werden vorliegend Scandium (21), Yttrium (39), Lanthan (57) und die 14 auf Lanthan (57) folgenden Elemente, nämlich Cer (58), Praseodym (59), Neodym (60), Promethium (61), Samarium (62), Europium (63), Gadolinium (64), Terbium (65), Dysprosium (66), Holmium (67), Erbium (68), Thulium (69), Ytterbium (70) und Lutetium (71), verstanden.

Die Legierungen der Elemente Magnesium, Eisen oder Wolfram sind so in ihrer Zusammensetzung zu wählen, dass sie biokorrodierbar sind. Als biokorrodierbar im Sinne der Erfindung werden Legierungen bezeichnet, bei denen in physiologischer Umgebung ein Abbau stattfindet, der letztendlich dazu führt, dass das gesamte Implantat oder der aus dem Werkstoff gebildete Teil des Implantates seine mechanische Integrität verliert. Als Prüfmedium zur Testung des Korrosionsverhaltens einer in Frage kommenden Legierung dient künstliches Plasma, wie es nach EN ISO 10993-15:2000 für Biokorrosionsuntersuchungen vorgeschrieben ist (Zusammensetzung NaCl 6,8 g/l, CaCl₂ 0,2 g/l, KCI 0,4 g/l, MgSO₄ 0,1 g/l, NaHCO₃ 2,2 g/l, Na₂HPO₄ 0,126 g/l, NaH₂PO₄ 0,026 g/l). Eine Probe der zu untersuchenden Legierung wird dazu in einem verschlossenen Probenbehälter mit einer definierten Menge des Prüfmediums bei 37 °C gelagert. In zeitlichen Abständen - abgestimmt auf das zu erwartende Korrosionsverhalten - von wenigen Stunden bis zu mehreren Monaten werden die Proben entnommen und in bekannter Weise auf Korrosionsspuren untersucht. Das künstliche Plasma nach EN ISO 10993-15:2000 entspricht einem blutähnlichen Medium und stellt damit eine Möglichkeit dar, eine physiologische Umgebung im Sinne der Erfindung reproduzierbar nachzustellen.

Ein Korrosionssystem besteht vorliegend aus dem korrodierenden metallischen Werkstoff sowie einem flüssigen Korrosionsmedium, das in seiner Zusammensetzung die Verhältnisse in physiologischer Umgebung nachstellt oder ein physiologisches Medium, insbesondere Blut ist. Werkstoffseitig beeinflussen die Korrosion Faktoren, wie die Zusammensetzung und Vorbehandlung der Legierung, mikro- und submikroskopische Inhomogenitäten, Randzoneneigenschaften, Temperatur- und Spannungszustand und insbesondere die Zusammensetzung einer die Oberfläche bedeckenden Schicht. Auf Seiten des Mediums wird der Korrosionsprozess durch Leitfähigkeit, Temperatur, Temperaturgradienten, Azidität, Volumen-Oberflächen-Verhältnis, Konzentrationsunterschied sowie Strömungsgeschwindigkeit beeinflusst.

Implantate im Sinne der Erfindung sind über ein chirurgisches Verfahren in den Körper eingebrachte Vorrichtungen und umfassen Befestigungselemente für Knochen, beispielsweise Schrauben, Platten oder Nägel, chirurgisches Nahtmaterial, Darmklammern, Gefäßclips, Prothesen im Bereich des Hart- und Weichgewebes und Ankerelemente für Elektroden, insbesondere von Schrittmachern oder Defibrillatoren.

Vorzugsweise ist das Implantat ein Stent. Stents herkömmlicher Bauart weisen eine filigrane Stützstruktur aus metallischen Streben auf, die zur Einbringung in den Körper zunächst in einem nicht-expandierten Zustand vorliegt und die am Ort der Applikation dann in einen expandierten Zustand aufgeweitet wird.

Nach einer bevorzugten Ausführungsform ist der Röntgenmarker ein Tantalcarbid oder ein Wolframcarbid, besonders bevorzugt TaC. Die genannten Werkstoffe zeichnen sich durch eine für den medizinischen Einsatz gute Röntgensichtbarkeit sowie ein gegenüber physiologischen Medien inertes Verhalten aus.

Der Röntgenmarker kann in massiver Ausführung als Vollmaterial vorliegen und mit geeigneten Halteelementen oder durch Kleben, Löten und Heften mit dem Implantat verbunden werden. Nach einer bevorzugten Ausführungsform liegt der Röntgenmarker jedoch als Pulver mit einer mittleren Teilchengröße im Bereich von 0,1 bis 20 Mikrometer vor, wobei das Pulver in eine organische biodegradierbare Trägermatrix eingebettet ist. Die organische Trägermatrix besteht im Wesentlichen aus einer organischen Verbindung, insbesondere aus einem Polymer. Der Vorteil liegt unter anderem in der Vereinfachung der Verarbeitung: Es kann eine Dispersion aus den beiden Komponenten organische Trägermatrix und Röntgenmarker-Pulver - gegebenenfalls unter Zusatz eines geeigneten Lösungsmittels - hergestellt werden, die sich über herkömmliche Beschichtungsverfahren auf das Implantat auftragen lässt oder als Füllmaterial für eine Kavität im Implantat dient. Nach dem Abbau der biokorrodierbaren Trägermatrix verbleibt das Röntgenmarker-Pulver und wird vermutlich aufgrund seiner geringen Teilchengröße in extrazellulären Vesikeln gespeichert. Es ist davon auszugehen, dass eine derartige Einlagerung des Materials Abstoßungsreaktionen mindert.

Nach einer ersten Variante besteht die biodegradierbare Trägermatrix ganz oder zu mindestens 80 Gew.% bezogen auf das Gesamtgewicht der Trägermatrix aus einem oder mehreren biodegradierbaren Polymeren. Insbesondere besteht die Trägermatrix aus einem Polylactid, z.B. Poly-L-Lactid. Nach einer zweiten Variante kann die biodegradierbare Trägermatrix ganz oder zu mindestens 80 Gew.% bezogen auf das Gesamtgewicht der Trägermatrix aus einem oder mehreren biodegradierbaren Fetten oder Ölen bestehen.

Eine weitere bevorzugte Ausführungsform mit einem pulverförmigen Röntgenmarker - die insbesondere auch mit den beiden vorgenannten bevorzugten Varianten der biodegradierbare Trägermatrix realisierbar ist - sieht vor, dass ein Gewichtsanteil des Röntgenmarkers bezogen auf das Gesamtgewicht aus Trägermatrix und Röntgenmarker im Bereich von 15 bis 90 Gew.% liegt. Bei der ersten Variante mit einer Trägermatrix aus einem biodegradierbaren Polymer liegt der Gewichtsanteil des Röntgenmarkers bezogen auf das Gesamtgewicht aus Trägermatrix und Röntgenmarker vorzugsweise im Bereich von 15 bis 90 Gew.%. Bei der zweiten Variante mit einer organischen biodegradierbaren Trägermatrix aus einem Fett oder Öl liegt der Gewichtsanteil des Röntgenmarkers bezogen auf das Gesamtgewicht aus Trägermatrix und Röntgenmarker vorzugsweise im Bereich von 80 bis 90 Gew.%. Hierdurch ist zum Einen sichergestellt, dass das Material eine ausreichende Röntgensichtbarkeit auch bei relativ geringen Materialdicken besitzt und zum Anderen noch eine Verarbeitung als Dispersion möglich ist.

Ein zweiter Aspekt der Erfindung liegt in der Bereitstellung eines medizinischen Implantats mit einem Röntgenmarker entsprechend den vorgenannten Ausführungen. Insbesondere ist dieses medizinische Implantat ein Stent, vorzugsweise ein Stent aus einer biokorrodierbaren Magnesiumlegierung.

Schließlich bezieht sich ein dritter Aspekt der Erfindung auf die Verwendung eines Borids oder Carbids der Elemente Tantal oder Wolfram als Röntgenmarker für medizinische Implantate.

Ein Stent aus der biokorrodierbaren Magnesiumlegierung WE43 (93 Gew.% Magnesium, 4 Gew.% Yttrium [W] und 3 Gew.% Seltenerdmetalle [E]) wurde wie folgt mit einem Röntgenmarker beschichtet:

### Ausführungsbeispiel 1 - TaC in polymerer Trägermatrix

Es wurde eine Dispersion aus einem PEG/PLGA-Copolymer (Diblock Copolymer aus Polyethylenglycol (PEG) und Poly(DL-lactid-co-glycolid) (PLGA) mit M_{w} 5.000; bei der Fa. Boehringer Ingelheim, Deutschland unter dem Handelsnamen Resomer RGP d 50155 erhältlich) und TaC-Pulver (bei der Fa. Chempur erhältlich) mit einer mittleren Teilchengröße von etwa 10 µm in Azeton angesetzt, wobei ein Gewichtanteil des TaC-Pulvers bezogen auf das Gesamtgewicht aus Copolymer und Röntgenmarker bei 75 Gew.% lag. Die Stentenden wurden in die durch Rühren homogenisierte Dispersion getaucht und anschließend an Luft getrocknet.

Die entstandenen Tropfen aus TaC/Resomer wiesen nach mehrmaligem Eintauchen eine Dicke von etwa 150 µm auf.

Ausführungsbeispiel 1 - TaC in einem Fett 90 Gew.% TaC-Pulver (Bezugsquelle wie Ausführungsbeispiel 1) wurden in 10 Gew.% hydrierten Sojaöl bei etwa 65°C beider Fa. Hees erhältlich) eingerührt und homogenisiert. Diese Suspension wurde dann in eine Kavität im Stent dispergiert.

## Patentansprüche

1. Röntgenmarker für medizinische Implantate aus einem biokorrodierbaren metallischen Werkstoff, wobei der Röntgenmarker ein Borid oder Carbid der Elemente Tantal oder Wolfram ist.

2. Röntgenmarker nach Anspruch 1, bei dem der biokorrodierbare metallische Werkstoff eine Legierung ausgewählt aus der Gruppe Magnesium, Eisen und Wolfram ist.

3. Röntgenmarker nach Anspruch 2, bei dem der biokorrodierbare metallische Werkstoff eine Magnesiumlegierung ist.

4. Röntgenmarker nach Anspruch 1, bei dem das Implantat ein Stent ist.

5. Röntgenmarker nach einem der vorhergehenden Ansprüche, bei dem der Röntgenmarker ein Tantalcarbid oder Wolframcarbid ist.

6. Röntgenmarker nach einem der vorhergehenden Ansprüche, bei dem der Röntgenmarker als Pulver mit einer mittleren Teilchengröße im Bereich von 0,1 - 20 µm vorliegt und das Pulver in eine biodegradierbare Trägermatrix eingebettet ist.

7. Röntgenmarker nach Anspruch 6, bei dem die Trägermatrix ganz oder zu mindestens 80 Gew.% bezogen auf das Gesamtgewicht der Trägermatrix aus einem oder mehreren biodegradierbaren Polymeren besteht.

8. Röntgenmarker nach Anspruch 6, **dadurch gekennzeichnet, dass** die Trägermatrix ganz oder zu mindestens 80 Gew.% bezogen auf das Gesamtgewicht der Trägermatrix aus einem oder mehreren biodegradierbaren Fetten oder Ölen besteht.

9. Röntgenmarker nach einem der Ansprüche 6 bis 8, bei dem ein Gewichtsanteil des Röntgenmarkers bezogen auf das Gesamtgewicht aus Trägermatrix und Röntgenmarker im Bereich von 15 bis 90 Gew.% liegt.

10. Medizinisches Implantat, mit einem Röntgenmarker nach einem der vorhergehenden Ansprüche.

11. Verwendung eines Borids oder Carbids der Elemente Tantal oder Wolfram als Röntgenmarker für medizinische Implantate.
